# FASCICULE DE BREVET EUROPEEN

(11) **EP 0 663 202 B1**
(45) Date de publication et mention de la délivrance du brevet: **29.07.1998**
(21) Numéro de dépôt: 95400086.5
(22) Date de dépôt: 17.01.1995
(51) Int. Cl.: A61K 7/032, A61K 7/48

(54) **Composition cosmétique pour le maquillage sous forme d'un mascara contenant au moins une cire et un pseudo-latex de dérivés de la cellulose**
Maskara kosmetische Zusammensetzungen, die wenigstens einen Wachs und einen Pseudo-Latex von Cellulosederivaten
Mascara make-up cosmetic compositions containing at least one wax and cellulose derivate pseudo-latex

(30) Priorité: 17.01.1994 FR 9400424
(43) Date de publication de la demande: 19.07.1995
(73) Titulaire: L'OREAL, 75008 Paris (FR)
(72) Inventeur: Mougin, Nathalie, F-75011 Paris (FR); Mondet, Jean, F-93700 Drancy (FR); Piot, Bertrand, F-92250 La Garenne-Colombes (FR); Junino, Alex, F-93190 Livry-Gargan (FR); Patraud, Jeanne, F-75013 Paris (FR)
(74) Mandataire: Stalla-Bourdillon, Bernard

(56) Documents cités:
- FR-A- 2 400 890
- US-A- 3 646 214
- DATABASE WPI Week 7850, Derwent Publications Ltd., London, GB; AN 78-90402A & JP-A-53 127 836

## Description

La présente invention a pour objet une composition cosmétique pour le maquillage notamment des cils, dite composition de mascara, contenant l'association d'au moins une cire et d'un pseudo-latex de dérivés de la cellulose.

Il est d'usage courant de réaliser des compositions de mascara contenant au moins une cire, toutefois, celle-ci n'est jamais utilisée seule, car le maquillage avec de telles compositions s'avère très médiocre conduisant à la formation sur les cils d'un film non homogène ce qui se traduit par la formation de pellicules craquantes, immédiatement après le séchage.

Afin d'y remédier, il a été proposé dans FR 83.09997 (2.528.699) et FR 84.17661 (2.573.305), l'utilisation conjointe d'au moins une cire et d'un polymère filmogène présent en solution dans la phase aqueuse.

Il a également été proposé dans WO/92/21316, des compositions de traitement des cheveux et des cils contenant l'association d'une silicone, d'un latex et d'un agent de mise en suspension du latex et de la silicone et/ou d'un épaississant.

Il a par ailleurs été proposé des compositions de mascara ne contenant pas de cire. Ainsi, il a été décrit dans JP 57-62216 (KOKAI), une composition de mascara aqueuse contenant comme agent filmogène un latex de synthèse.

Si ces compositions ont permis une certaine amélioration de la qualité des produits de maquillage, elles ne procurent cependant pas un allongement satisfaisant des cils et sont en outre difficilement éliminables à l'eau. On a maintenant constaté de façon surprenante et inattendue que l'association d'au moins une cire et d'un pseudo-latex particulier constitué de particules d'un polymère dérivé de la cellulose permettait d'obtenir des compositions de mascara augmentant significativement l'allongement et le recourbement des cils, et étant éliminables à l'eau tout en ayant d'excellentes qualités cosmétiques.

On rappelle que par l'expression "pseudo-latex", on désigne une suspension constituée de particules généralement sphériques d'un polymère, celles-ci étant obtenues par dispersion du polymère dans une phase aqueuse appropriée.

L'expression "pseudo-latex" ne doit pas être confondue avec l'expression "latex" ou "latex synthétique" qui est également une suspension constituée de particules d'un polymère qui sont obtenues directement par polymérisation d'un ou plusieurs monomères dans une phase aqueuse appropriée.

Plus précisément, la présente invention a pour objet une composition de mascara contenant en mélange,
a) un pseudo-latex constitué de particules, de diamètre moyen compris entre 10 et 300 nm, d'un polymère filmogène dérivé de la cellulose, insoluble dans l'eau, choisi parmi les éthers de cellulose, les esters de cellulose non-ioniques et les esters de cellulose anioniques à fonctions acides carboxyliques, lesdites fonctions acides carboxyliques étant neutralisées à un taux de neutralisation compris entre 10 et 80 % à l'aide d'un agent basique non volatil, et
b) au moins une cire ayant un point de fusion compris entre 60°C et 110°C, et de préférence entre 65°C et 100°C.

Selon l'invention, la composition de mascara contient en mélange de 0,8 à 20 %, et de préférence de 1 à 10 % en poids de matière sèche, du pseudo-latex et de 2 à 40 % en poids d'au moins une cire, ces pourcentages étant exprimés par rapport au poids total de la composition du mascara.

Selon l'invention, le rapport en poids entre le pseudo-latex exprimé en poids de matière sèche et la cire est de préférence compris entre 0,025 et 2, et plus particulièrement entre 0,5 et 1.

La taille moyenne des particules du pseudo-latex est de préférence inférieure ou égale à 250 nm.

La polydispersité en taille des particules est relativement faible, celle-ci mesurée en diffusion quasi-élastique de lumière est généralement comprise entre 0,05 et 0,40 et de préférence inférieure à 0,35.

La concentration en poids du polymère filmogène sous forme de particules dans le pseudo-latex est généralement comprise entre 5 et 50 % et de préférence entre 10 et 25 % par rapport au poids total du pseudo-latex.

Les polymères filmogènes dérivés de la cellulose insolubles dans l'eau tels que définis ci-dessus ont de préférence un poids moléculaire moyen compris entre 2.000 et 700.000, et en particulier compris entre 5.000 et 500.000, mesuré par exemple en chromatographie d'exclusion stérique.

Parmi les éthers de cellulose, insolubles dans l'eau, utilisables comme polymère filmogène selon l'invention, on peut citer notamment les éthylcelluloses et en particulier celles commercialisées sous les dénominations de "Ethocel" par la Société Dow Chemical.

Parmi les esters de cellulose non ioniques, insolubles dans l'eau, utilisables comme polymère filmogène selon l'invention, on peut citer notamment les acétates de cellulose, les propionates de cellulose, les butyrates de cellulose, les acéto-propionates de cellulose et les acéto-butyrates de cellulose.

Les pseudo-latex à base des polymères filmogènes décrits précédemment sont obtenus selon les méthodes connues de préparation des pseudo-latex.

Le procédé général de préparation des pseudo-latex consiste à dissoudre le polymère filmogène, insoluble dans l'eau, dans un solvant organique, miscible ou partiellement miscible dans l'eau, à disperser sous agitation la solution ainsi obtenue dans de l'eau à une température comprise entre la température ambiante et 70°C environ, à évaporer sous pression réduite, et de préférence sous léger chauffage, le solvant organique jusqu'à son élimination totale. On obtient ainsi un pseudo-latex, c'est-à-dire une suspension aqueuse de particules, celles-ci ayant généralement une taille inférieure au µm.

Le solvant organique utilisé doit être un solvant volatil ou un mélange de tels solvants présentant un point d'ébullition inférieur à celui de l'eau.

Le solvant organique tel que défini ci-dessus est de préférence choisi parmi l'acétone, la méthyléthylcétone, le tétrahydrofuranne, le 1,2-dichloroéthane, l'acétate de méthyle, l'acétate d'éthyle, l'isopropanol et l'éthanol.

Selon ce procédé général, on utilise de préférence en outre un agent dispersant choisi parmi un agent tensio-actif, un mélange d'agents tensio-actifs ou un polymère colloïde protecteur hydrosoluble ou encore un mélange agent tensio-actif/polymère colloïde protecteur hydrosoluble, en vue d'améliorer la stabilisation des particules.

Les agents tensio-actifs utilisables selon l'invention peuvent être du type anionique, non-ionique, cationique ou amphotère. On utilise toutefois de préférence des agents tensio-actifs de type anionique ou non-ionique.

Parmi ceux-ci, ont peut citer notamment le laurylsulfate de sodium.

On peut en outre associer l'agent tensio-actif utilisé à un costabilisant soluble dans la phase organique tel que l'alcool cétylique.

Comme polymères colloïdes protecteurs hydrosolubles, on peut citer notamment l'alcool polyvinylique, la gomme arabique et les polymères séquencés polyoxyéthylène/polyoxypropylène.

Lorsque le polymère filmogène dérivé de la cellulose, insoluble dans l'eau est un ester de cellulose anionique à fonctions acides carboxyliques, celui-ci est choisi parmi : l'acétophtalate de cellulose, le succinate d'acétate de cellulose, le succinate de propionate de cellulose, le succinate de butyrate de cellulose, le succinate d'acétopropionate de cellulose, le succinate d'acétobutyrate de cellulose, le trimellilate d'acétate de cellulose, le trimellilate de butyrate de cellulose, le trimellilate de propionate de cellulose, le trimellilate d'acétopropionate de cellulose et le trimellilate d'acétobutyrate de cellulose.

Les pseudo-latex constitués par les esters de cellulose anioniques à fonctions acides carboxyliques sont obtenus selon le procédé général tel que décrit précédemment sous réserve toutefois de certaines particularités.

Les esters de cellulose anioniques à fonctions acides carboxyliques tels que définis ci-dessus ne peuvent être utilisés tels quels dans la préparation des pseudo-latex mais doivent être neutralisés à un taux de neutralisation inférieur à 100 % en vue d'éviter leur totale solubilisation dans l'eau.

Par une neutralisation partielle des polymères, on a constaté qu'il était possible d'obtenir des pseudo-latex particulièrement stables en l'absence de stabilisant hydrophile ou d'agent tensio-actif ou encore de polymère colloïde protecteur.

Le taux de neutralisation des polymères filmogènes à fonctions acides carboxyliques doit en outre être parfaitement déterminé de telle sorte qu'ils restent insolubles dans l'eau tout en étant solubles dans le solvant organique.

Il va de soi que le taux limite supérieur de neutralisation qu'il conviendra de ne pas excéder pour que le polymère reste insoluble dans l'eau sera fonction de la nature de chaque ester de cellulose anionique à fonctions acides carboxyliques. De façon générale, ce taux de neutralisation est généralement compris entre 30 et 80 %, et de préférence entre 40 et 70 % si le polymère a moins de 2 meq/g de fonctions acides carboxyliques et entre 10 et 50 %, de préférence entre 10 et 40 %, si le polymère a plus de 2 meq/g de fonctions acides carboxyliques.

Selon l'invention la neutralisation des fonctions acides carboxyliques est réalisée à l'aide d'un agent basique non volatil choisi par exemple parmi une base minérale telle que la soude ou la potasse ou parmi un aminoalcool choisi dans le groupe constitué par l'amino-2 méthyl-2 propanol-1 (AMP), la triéthanolamine, la triisopropanolamine (TIPA), la monoéthanolamine, la diéthanolamine, la tri [(hydroxy-2) propyl-1] amine, l'amino-2 méthyl-2 propanediol-1,3 (AMPD) et l'amino-2 hydroxyméthyl-2 propanediol-1,3.

Dans la préparation du pseudo-latex, utilisé dans les compositions selon l'invention, la neutralisation des fonctions acides carboxyliques de l'ester de cellulose anionique est réalisée *in situ* dans la solution du polymère, dans le solvant organique, par addition de la quantité déterminée du composé basique non volatil.

Le solvant organique utilisé est tel que défini précédemment dans le procédé général.

Après l'obtention de la solution d'ester de cellulose anionique partiellement neutralisé dans le solvant organique, on procède alors à la préparation d'une émulsion en versant sous agitation, à la solution organique obtenue, une quantité appropriée d'eau contenant éventuellement un agent anti-mousse dont le rôle sera de faciliter l'évaporation ultérieure de la phase organique.

Selon une variante du procédé tel que défini ci-dessus, la neutralisation des fonctions acides carboxyliques du polymère en solution dans le solvant organique peut être réalisée lors de la formation de l'émulsion en versant une solution aqueuse contenant la quantité requise du composé basique non volatil.

Lors de la formation de l'émulsion, l'agitation est de préférence réalisée à l'aide d'un disperseur cisaillant de type Moritz, Ultra-Turrax ou Raineri et équipé de pales défloculantes.

L'émulsion ainsi obtenue est particulièrement stable dans la mesure où les groupes carboxylates du polymère se placent à l'interface avec l'eau et protègent les gouttelettes de la coalescence par répulsion électrostatique.

Après formation de l'émulsion, on procède à l'élimination du solvant organique sous vide partiel tel que décrit précédemment.

Selon ce mode de réalisation de l'invention, on obtient un pseudo-latex exempt de tout agent tensio-actif ou autre stabilisant hydrophile et particulièrement stable.

On peut introduire dans les pseudo-latex utilisés dans les compositions selon l'invention, en vue d'améliorer leur propriétés cosmétiques et mécaniques, un agent plastifiant en une proportion comprise entre 5 et 90 % et de préférence entre 10 et 80 % en poids par rapport au poids du polymère filmogène, ledit agent se distribuant selon son coefficient de partage entre les particules et la phase aqueuse du pseudo-latex.

L'agent plastifiant qui peut être du type hydrophile ou hydrophobe, est de préférence introduit en mélange dans le solvant organique lors de la préparation du pseudo-latex, et notamment lorsqu'il est du type hydrophobe.

Lorsque l'agent plastifiant est du type hydrophile, il peut être introduit après formation du pseudo-latex dans la phase aqueuse.

Parmi les agents plastifiants pouvant être utilisés dans les compositions selon l'invention, on peut citer :
- les "Carbitols" de la Société Union Carbide à savoir le "Carbitol" ou diéthylène glycol éthyléther, le "méthyl Carbitol" ou diéthylène glycol méthyléther, le "butyl Carbitol" ou diéthylène glycol butyléther ou encore l'"hexyl Carbitol" ou diéthylène glycol hexyléther,
- les "Cellosolves" de la Société Union Carbide à savoir le "Cellosolve" ou éthylène glycol éthyléther, le "butyl Cellosolve" ou éthylène glycol butyléther, l'"hexyl Cellosolve" ou éthylène glycol hexyléther,
- les dérivés de propylène glycol et en particulier le propylène glycol phényléther, le propylène glycol diacétate, le dipropylène glycol butyléther, le tripropylène glycol butyléther, ainsi que les "Dowanols" de la Société Dow Chemical à savoir le "Dowanol PM" ou propylène glycol méthyléther, le "Dowanol DPM" ou dipropylène glycol méthyléther et le "Dowanol TPM" ou tripropylène glycol méthyléther.

On peut encore citer :
- le diéthylène glycol méthyléther ou "Dowanol DM" de la Société Dow Chemical,
- l'huile de ricin oxyéthylénée à 40 moles d'oxyde d'éthylène telle que celle vendue par la Société Rhône Poulenc sous la dénomination de "Mulgofen EL-719",
- l'alcool benzylique,
- le citrate de triéthyle vendu par la Société Pfizer sous la dénomination de "Citroflex-2",
- le 1,3-butylène glycol,
- les phtalates et adipates de diéthyle, de dibutyle et de diisopropyle,
- les tartrates de diéthyle et de dibutyle,
- les phosphates de diéthyle, de dibutyle et de diéthyl-2 hexyle, et
- les esters de glycérol tels que le diacétate de glycérol (diacétine) et le triacétate de glycérol (triacétine).

On utilise de préférence un agent plastifiant choisi dans le groupe constitué par le dipropylène glycol méthyléther, le tripropylène glycol méthyléther, l'adipate de diéthyle, l'adipate de diisopropyle, et le triacétate de glycérol.

Les cires utilisées dans les compositions de mascara selon l'invention sont choisies parmi les cires, solides et rigides, d'origine animale, végétale, minérale ou de synthèse et leurs mélanges.

La dureté de ces cires, mesurée par la méthode de pénétration d'une aiguille est généralement comprise entre 3 et 40.

Cette méthode décrite dans les normes NFT 004 et ASTM D5, normes respectivement française et américaine, consiste à mesurer, à une température de 25°C, la profondeur de pénétration, exprimée en dixième de millimètre, d'une aiguille normalisée (pesant 2,5 g et placée dans un porte-aiguille pesant 47,5 g, soit au total 50 g) placée sur la cire pendant 5 secondes.

Parmi les cires animales, on peut citer la cire d'abeilles, la cire de lanoline, et les cires d'insectes de Chine.

Parmi les cires végétales, on peut notamment citer la cire de riz, la cire de carnauba, la cire de candelilla, la cire d'ouricurry, la cire de fibres de liège, la cire de canne à sucre, la cire du Japon et la cire de sumac.

Parmi les cires minérales, on peut notamment citer la cire de montan, les cires microcristallines, les paraffines et l'ozokérite.

Parmi les cires de synthèse, on peut notamment citer les cires de polyéthylène, les cires obtenues par la synthèse de Fisher et Tropsch et les copolymères cireux ainsi que leurs esters.

On peut également utiliser dans les compositions selon l'invention, les cires obtenues par hydrogénation catalytique d'huiles animales ou végétales ayant des chaînes grasses, linéaires, ou ramifiées, en C₈-C₃₂.

Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.

La viscosité des compositions de mascara selon l'invention, exprimée en unités de déviation et mesurée au Contraves TV à l'aide du mobile 4 pendant 10 minutes à 25°C, est généralement comprise entre 21 et 85 unités de déviation et de préférence entre 25 et 64 unités de déviation.

Les compositions de mascara selon l'invention peuvent en outre contenir des pigments.

Ces pigments peuvent être organiques ou minéraux ou peuvent être également des pigments nacrés. De tels pigments sont bien connus et sont en particulier décrits dans FR 83.09997 (2.528.699).

La proportion en pigments dans les compositions de mascara selon l'invention est généralement comprise entre 3 et 25 % en poids par rapport au poids total de la composition suivant la coloration et l'intensité de coloration recherchées.

Les compositions de mascara selon l'invention peuvent se présenter sous différentes formes. Elles peuvent en particulier se présenter sous forme d'émulsions huile-dans-eau ou eau-dans-huile ou sous forme de dispersions.

Selon une forme de réalisation préférée des compositions de mascara selon l'invention, celles-ci se présentent sous forme d'émulsions contenant au moins un agent tensio-actif anionique ou non ionique en une proportion comprise entre 2 et 30 % en poids par rapport au poids total de la composition.

Parmi les agents tensio-actifs anioniques pouvant être utilisés seuls ou en mélange, on peut citer notamment les sels alcalins, les sels d'ammonium, les sels d'amine ou les sels d'amino-alcool des composés suivants :
- les alcoylsulfates, alcoyléther sulfates, alcoylamide sulfates, éther sulfates, alcoylarylpolyéther sulfates, monoglycéride sulfates,
- les alcoylsulfonates, alcoylamide sulfonates, alcoylarylsulfonates, α-oléfines sulfonates, paraffines sulfonates,
- les alcoylsulfosuccinates, alcoyléther sulfosuccinates, les alcoylamide sulfosuccinates,
- les alcoylsulfosuccinamates,
- les alcoylsulfoacétates, alcoylpolyglycérol carboxylates,
- les alcoylphosphates/alcoyléther phosphates,
- les alcoylsarcosinates, alcoylpolypeptidates, alcoylamidopolypeptidates, les alcoyliséthionates, et alcoyltaurates.

On entend par le terme alcoyle utilisé ci-dessus une chaîne hydrocarbonée ayant généralement de 12 à 18 atomes de carbone.

On peut citer également comme agents tensio-actifs anioniques utilisables dans les compositions selon l'invention des sels d'acides gras, tels que ceux de l'acide oléïque, ricinoléique, palmitique, stéarique, les acides d'huile de coprah ou d'huile de coprah hydrogénée, et en particulier les sels d'amines tels que les stéarates d'amines.

On peut encore citer comme agents tensio-actifs anioniques les acyl lactylates dont le radical acyle comprend de 8 à 20 atomes de carbone, et les acides carboxyliques d'éthers polyglycoliques répondant à la formule :

R₄-(OCH₂-CH₂)ₙ-OCH₂-COOH

dans laquelle :
R₄ représente un radical alkyle linéaire ayant de 12 à 18 atomes de carbone et n est un nombre entier compris entre 5 et 15, et les sels desdits acides. On utilise de préférence comme agent tensio-actif anionique des stéarates d'amines.

Parmi les agents tensio-actifs non ioniques pouvant être utilisés seuls ou en mélange dans les compositions de mascara selon l'invention, on peut citer notamment les alcools, les alcoylphénols et les acides gras polyéthoxylés, polypropoxylés ou polyglycérolés à chaîne grasse ayant de 8 à 18 atomes de carbone.

On peut également citer des copolymères d'oxydes d'éthylène et de propylène, des condensats d'oxydes d'éthylène et de propylène sur des alcools gras, des amides gras polyéthoxylés, des amines grasses polyéthoxylées, des éthanolamides, des esters d'acides gras de glycol, des esters d'acides gras du sorbitan oxyéthylénés ou non, des esters d'acides gras du saccharose, des esters d'acides gras des polyéthylèneglycols, des triesters phosphoriques et des esters d'acides gras de dérivés du glucose.

On peut également citer les produits de condensation d'un monoalcool, d'un alpha-diol, d'un alcoylphénol, d'un amide ou d'un diglycolamide avec le glycidol ou un précurseur du glycidol tel que décrit dans le brevet français FR 71.17206 (2.091.516), de formule :

R₅-CHOH-CH₂-O-(CH₂-CHOH-CH₂-O)ₚ-H

dans laquelle :
R₅ représente un radical aliphatique, cycloaliphatique, ou arylaliphatique ayant de préférence entre 7 et 21 atomes de carbone, les chaînes aliphatiques pouvant comporter des groupements éther, thioéther ou hydroxyméthylène, et p est un nombre entier compris entre 1 et 10.

On peut en outre citer les composés décrits dans le brevet FR 1.477.048 de formule :

R₆O-[C₂H₃O-(CH₂OH)]_{q}-H

dans laquelle :
R₆ représente un radical alcoyle, alcényle ou alcoylaryle, et q est une valeur statistique comprise entre 1 et 10.

On peut encore citer les composés décrits dans le brevet français FR 76.31975 (2.328.763) de formule :

R₇CONH-CH₂-CH₂O-CH₂-CH₂O-(CH₂-CHOH-CH₂-O)ᵣ-H

dans laquelle :
R₇ représente un radical aliphatique linéaire ou ramifié, saturé ou insaturé, pouvant comporter éventuellement un ou plusieurs groupement(s) hydroxyle(s), ayant entre 8 et 30 atomes de carbone, d'origine naturelle ou synthétique, et r est un nombre entier ou décimal compris entre 1 et 5 et désigne le degré de condensation moyen.

On utilise de préférence comme agent tensio-actif non ionique un mélange d'huile(s) et/ou d'alcool gras ou bien d'alcools polyéthoxylés ou polyglycérolés tels que les alcools stéarylique ou cétylstéarylique polyéthoxylés.

Les compositions de mascara selon l'invention peuvent comprendre en outre au moins un additif conventionnel choisi parmi un adoucissant, un conservateur, un agent séquestrant, un parfum, un agent épaississant, une huile, une silicone, un agent de cohésion, un agent alcalinisant ou acidifiant, un polymère hydrosoluble et une charge.

Les agents épaississants utilisables dans les compositions de mascara selon l'invention peuvent être d'origine naturelle ou synthétique.

Parmi les agents épaississants d'origine naturelle, on peut citer notamment les gommes de diverses sortes telles que les gommes arabique, de guar ou de caroube.

Parmi les agents épaississants d'origine synthétique, on peut citer notamment les dérivés cellulosiques hydrosolubles, les dérivés d'éthers de cellulose possédant des groupes ammonium quaternaires, les dérivés de l'amidon, les polysaccharides cationiques, les sels de polymères acryliques ou méthacryliques, les polyènes et les polysiloxanes.

Selon un mode de réalisation préféré, l'agent épaississant d'origine synthétique utilisé est un dérivé cellulosique hydrosoluble choisi parmi les méthylcelluloses, les hydroxyéthylcelluloses, les hydroxypropylméthylcelluloses, les carboxyméthylcelluloses et leurs mélanges.

On peut également obtenir un épaississement des compositions de mascara selon l'invention par adjonction d'un mélange de polyéthylène glycol et de stéarate et/ou de distéarate de polyéthylène glycol ou d'un mélange d'esters phosphoriques et d'amides gras.

Parmi les polymères hydrosolubles pouvant être utilisés dans les compositions de mascara selon l'invention, on peut citer notamment les dérivés de protéines d'origine animale ou végétale et plus particulièrement les dérivés de kératine tels que les hydrolysats de kératine et les kératines sulfoniques, la polyvinylpyrrolidone, les copolymères vinyliques tels que le copolymère de l'éther méthylvinylique et de l'anhydride maléïque ou le copolymère de l'acétate de vinyle et de l'acide crotonique, les glycoaminoglycanes, l'acide hyaluronique et ses dérivés et l'acide désoxyribonucléique et ses sels.

Parmi les charges pouvant être utilisées dans les compositions de mascara selon l'invention, on peut citer notamment celles décrites dans FR 91.10791 (2.680.681).

On va maintenant donner à titre d'illustration plusieurs exemples de préparation des pseudo-latex ainsi que des exemples de mascara selon l'invention.

### EXEMPLE DE PREPARATION DE PSEUDO-LATEX

### EXEMPLE I : Préparation du pseudo-latex du polymère succinate d'acétobutyrate de cellulose ("CAB SU 160" de la Société Eastman) neutralisé à 30 % d'après sonindice d'acide

30 g du polymère "CAB SU 160" (teneur en radicaux succinate : 9 % en poids ; indice d'acide : 60) sont additionnés petit à petit sous agitation à une solution homogène de 154,2 g d'acétone, 0,858 g d'amino-2 méthyl-2 propanol-1 (quantité correspondant à 30 % de neutralisation d'après l'indice d'acide) et 15 g d'adipate de diéthyle.

Après agitation à température ambiante pendant 30 minutes, la dissolution du polymère est totale.

A la phase organique ainsi obtenue, on ajoute en 15 minutes environ, sous agitation à l'aide d'un disperseur cisaillant du type Moritz à 2500 tr/min, une phase aqueuse pour réaliser l'émulsion, celle-ci étant constituée de 154 g d'eau permutée.

Après la fin de l'addition de la phase aqueuse, à température ambiante, on poursuit l'agitation pendant 10 à 15 minutes à 3000 tr/min, ce qui permet de conduire à l'obtention d'une émulsion translucide et stable.

On procède alors à la concentration à l'aide d'un évaporateur rotatif sous vide partiel à une température inférieure à 45°C. Après élimination totale de l'acétone, on obtient une suspension stable dont la concentration en polymère est de 17 % en poids par rapport au poids total de la dispersion.

La taille des particules a été mesurée en diffusion quasi-élastique de lumière au Coulteur modèle N4 et a donné les résultats suivants :
- Taille moyenne des particules : 101 nm
- Facteur de polydispersité : 0,13.

### EXEMPLE II : Préparation du pseudo-latex du polymère d'éthylcellulose ("Ethocel 10" de la Société Dow Chemical) stabilisé par un agent tensio-actif.

40 g du polymère "Ethocel 10" sont additionnés petit à petit à une solution homogène de 759 g de 1,2-dichloroéthane et de 1 g d'alcool cétylique. On agite à l'aide d'un disperseur cisaillant du type Moritz à 3000 tr/min, à température ambiante, jusqu'à dissolution totale du polymère, soit pendant environ 30 minutes.

A la phase organique ainsi obtenue, on ajoute en environ 30 minutes, sous agitation, une phase aqueuse pour réaliser l'émulsion, celle-ci étant constituée de 759 g d'eau permutée et de 1 g de laurylsulfate de sodium.

Après la fin de l'addition de la phase aqueuse, à température ambiante, on poursuit l'agitation pendant 30 minutes environ, ce qui permet de conduire à l'obtention d'une émulsion relativement grossière. On procède alors à l'homogénéisation de l'émulsion à l'aide d'un homogénéiseur haute pression du type Soavib & Figli, modèle OBL n° 2032, sous une pression de 75.10⁶ Pa. Après trois passages, on obtient une émulsion fine et homogène.

On procède alors à la concentration à l'aide d'un évaporateur rotatif sous vide partiel à une température inférieure ou égale à 50°C. Après élimination totale du 1,2-dicholoroéthane, on obtient une suspension stable dont la concentration en polymère est de 20 % en poids par rapport au poids total de la suspension.

La taille des particules a été mesurée en diffusion quasi-élastique de lumière au Coulteur modèle N4 et a donné les résultats suivants :
- Taille moyenne des particules : 135 nm
- Facteur de polydispersité : 0,09.

On peut en outre plastifier le pseudo-latex d'éthylcellulose obtenu précédemment. A 200 g de la suspension obtenue précédemment, on ajoute petit à petit 20 g d'adipate de diéthyle sous légère agitation magnétique. On obtient ainsi une suspension stable, fine, homogène et peu visqueuse.

### EXEMPLE III : Préparation du pseudo-latex d'acétate de cellulose ("AC 398-10" de la Société Eastman) plastifié et stabilisé par un agent tensio-actif

150 g d'acétate de cellulose "AC 398-10" sont additionnés petit à petit sous agitation dans une solution homogène de 669 g d'acétate d'éthyle, 1561 g de méthyléthylcétone et de 120 g de triacétate de glycérol.

On agite à 3000 tr/min à l'aide d'un disperseur de type Moritz, à température ambiante, jusqu'à dissolution totale du polymère, soit pendant environ 30 minutes.

A la phase organique ainsi obtenue, on ajoute en 30 minutes environ, une phase aqueuse pour réaliser l'émulsion, celle-ci étant constituée de 7,5 g de laurylsulfate de sodium dissous dans 2903 g d'eau permutée.

Après la fin de l'addition de la phase aqueuse, à température ambiante, on poursuit l'agitation pendant 30 minutes environ à 3000 tr/min, ce qui permet de conduire à l'obtention d'une émulsion grossière.

On procède alors à l'homogénéisation de l'émulsion selon le même procédé que décrit à l'exemple II.

Puis, on concentre la solution à l'aide d'un évaporateur rotatif sous vide partiel à une température inférieure à 45°C. On obtient alors un pseudo-latex plastifié, stable et homogène dont la concentration en extrait sec par rapport au poids total du pseudo-latex est de 14,2 % en poids et dont la concentration en polymère dérivé de cellulose insoluble dans l'eau est de 10,2 % en poids.

La taille des particules a été mesurée en diffusion quasi-élastique de lumière au Coulteur modèle N4 et a donné les résultats suivants :
- Taille moyenne des particules : 138 nm
- Facteur de polydispersité : 0,10.

### EXEMPLES DE MASCARA

### EXEMPLE 1 : Mascara crème

On prépare la composition de mascara suivante :

| *PARTIE A* | |
|---|---|
| - Stéarate de triéthanolamine | 11,5 g |
| - Cire d'abeilles | 7,0 g |
| - Cire de carnauba | 4,1 g |
| - Paraffine | 11,4 g |

| *PARTIE B* | |
|---|---|
| - Oxyde de fer noir | 5,5 g |

| *PARTIE C* | |
|---|---|
| - Gomme arabique | 4,5 g |
| - Hydroxyéthylcellulose commercialisée sous la dénomination de "Cellosize QP" par la Société Amerchol | 0,16 g |

| *PARTIE D* | |
|---|---|
| - Pseudo-latex de l'exemple I | 8,8 g |
| - Conservateurs qs | |
| - Eau qsp | 100 g |

Ce mascara est obtenu en portant les ingrédients de la partie *A* à 85°C, à laquelle on ajoute la partie *B* et l'on agite à l'aide d'une turbine.

On fait ensuite bouillir l'eau de la préparation, ajoute les conservateurs, puis à 85°C, les ingrédients de la partie *C*.

On ajoute alors la phase aqueuse obtenue (85°C) à la partie *A* (80°C) sous agitation à l'aide d'une turbine (émulsification à 30°C) puis on ajoute enfin le pseudo-latex de la partie *D* et agite à l'aide d'une pale.

Lorsque l'on utilise le mascara ainsi obtenu, par application sur les cils, ceux-ci paraissent plus longs et présentent une courbure prononcée. En outre, le démaquillage peut être aisément réalisé à l'eau.

### EXEMPLE 2 : Mascara

On prépare, selon le même mode opératoire qu'à l'exemple 1, un mascara ayant la composition suivante :

| *PARTIE A* | |
|---|---|
| - Stéarate de triéthanolamine | 11,5 g |
| - Cire d'abeilles | 7,0 g |
| - Cire de carnauba | 4,1 g |
| - Paraffine | 11,4 g |

| *PARTIE B* | |
|---|---|
| - Oxyde de fer noir | 5,0 g |

| *PARTIE C* | |
|---|---|
| - Gomme arabique | 4,5 g |
| - Hydroxyéthylcellulose commercialisée sous la dénomination de "Cellosize QP" par la Société Amerchol | 0,16 g |
| - Hydrolysat de kératine commercialisé sous la dénomination de "Kerasol" par la Société Croda | 2,0 g |

| *PARTIE D* | |
|---|---|
| - Pseudo-latex de l'exemple III | 14,1 g |
| - Conservateurs qs | |
| - Eau qsp | 100 g |

### EXEMPLE 3 : Mascara

On prépare, selon le même mode opératoire qu'à l'exemple 1, un mascara ayant la composition suivante :

| *PARTIE A* | |
|---|---|
| - Stéarate de glycéryle commercialisé sous la dénomination de "Geleol" par la Société Gattefosse | 3,0 g |
| - Stéarate de triéthanolamine | 8,0 g |
| - Cire d'abeilles | 7,0 g |
| - Cire de carnauba | 2,0 g |
| - Paraffine | 10,0 g |
| - Cire de candelilla | 4,0 g |

| *PARTIE B* | |
|---|---|
| - Noir de charbon | 2,5 g |

| *PARTIE C* | |
|---|---|
| - Gomme arabique | 4,5 g |
| - Hydroxyéthylcellulose commercialisée sous la dénomination de "Cellosize QP" par la Société Amerchol | 0,2 g |

| *PARTIE D* | |
|---|---|
| - Pseudo-latex de l'exemple II | 12,5 g |
| - Conservateurs qs | |
| - Eau qsp | 100 g |

## Revendications

1. Composition de mascara caractérisée par le fait qu'elle contient en mélange :
a) un pseudo-latex constitué de particules, de diamètre moyen compris entre 10 et 300 nm, d'un polymère filmogène dérivé de la cellulose, insoluble dans l'eau, choisi parmi les éthers de cellulose, les esters de cellulose non-ioniques et les esters de cellulose anioniques à fonctions acides carboxyliques, lesdites fonctions acides carboxyliques étant neutralisées à un taux de neutralisation compris entre 10 et 80 % à l'aide d'un agent basique non volatil, et
b) au moins une cire ayant un point de fusion compris entre 60°C et 110°C, et de préférence entre 65°C et 100°C.

2. Composition de mascara selon la revendication 1, caractérisée par le fait qu'elle contient de 0,8 à 20 % en poids de matière sèche du pseudo-latex et de 2 à 40 % en poids d'au moins une cire par rapport au poids total de ladite composition.

3. Composition de mascara selon la revendication 1 ou 2, caractérisée par le fait que le rapport en poids entre le pseudo-latex exprimé en poids de matière sèche et la cire est compris entre 0,025 et 2.

4. Composition de mascara selon l'une quelconque des revendications précédentes, caractérisée par le fait que le polymère filmogène a un poids moléculaire moyen compris entre 2.000 et 700.000 et de préférence compris entre 5.000 et 500.000.

5. Composition de mascara selon l'une quelconque des revendications précédentes, caractérisée par le fait que ledit polymère filmogène est un éther de cellulose choisi parmi les éthylcelluloses.

6. Composition de mascara selon l'une quelconque des revendications 1 à 4, caractérisée par le fait que ledit polymère filmogène est un ester de cellulose non-ionique choisi parmi les acétates de cellulose, les propionates de cellulose, les butyrates de cellulose, les acéto-propionates de cellulose et les acéto-butyrates de cellulose.

7. Composition de mascara selon l'une quelconque des revendications 1 à 4, caractérisée par le fait que ledit polymère filmogène est un ester de cellulose anionique à fonction acides carboxyliques choisi parmi l'acétophtalate de cellulose, le succinate d'acétate de cellulose, le succinate de propionate de cellulose, le succinate de butyrate de cellulose, le succinate d'acétopropionate de cellulose, le succinate d'acétobutyrate de cellulose, le trimellilate d'acétate de cellulose, le trimellilate de propionate de cellulose, le trimellilate de butyrate de cellulose, le trimellilate d'acétopropionate de cellulose et le trimellilate d'acétobutyrate de cellulose.

8. Composition de mascara selon l'une quelconque des revendications 1 à 4 et 7, caractérisée par le fait que les fonctions acides carboxyliques de l'ester de cellulose anionique sont neutralisées à l'aide d'un agent basique non volatil choisi parmi : la soude, la potasse, l'amino-2 méthyl-2 propanol-1, la triéthanolamine, la triisopropanolamine, la monoéthanolamine, la diéthanolamine, la tri[(hydroxy-2) propyl-1] amine, l'amino-2 méthyl-2 propanediol-1,3 et l'amino-2 hydroxyméthyl-2 propanediol-1,3.

9. Composition de mascara selon l'une quelconque des revendications précédentes caractérisée par le fait que le pseudo-latex contient un agent plastifiant en une proportion comprise entre 5 et 90 % en poids par rapport au poids du polymère filmogène, ledit agent plastifiant étant distribué selon son coefficient de partage entre les particules et la phase aqueuse du pseudo-latex.

10. Composition de mascara selon l'une quelconque des revendications précédentes caractérisée par le fait que ladite cire est choisie dans le groupe constitué par la cire d'abeilles, la cire de lanoline, la cire d'insectes de Chine, la cire de riz, la cire de carnauba, la cire de candelilla, la cire d'ouricurry, la cire de fibres de liège, la cire de canne à sucre, la cire du Japon, la cire de sumac, la cire de montan, les cires microcristallines, les paraffines, l'ozokérite, les cires de polyéthylène et les huiles hydrogénées.

11. Composition de mascara selon l'une quelconque des revendications précédentes caractérisée par le fait qu'elle contient en outre des pigments en une proportion comprise entre 3 et 25 % en poids par rapport au poids total de la composition.

12. Composition de mascara selon l'une quelconque des revendications précédentes caractérisé par le fait qu'elle se présente sous forme d'une émulsion huile-dans-eau ou eau-dans-huile ou d'une suspension.

13. Composition de mascara selon la revendication 12, caractérisée par le fait qu'elle se présente sous forme d'une émulsion contenant au moins un agent tensio-actif anionique ou non-ionique en une proportion comprise entre 2 et 30 % en poids par rapport au poids total de la composition.

14. Composition de mascara selon l'une quelconque des revendications précédentes caractérisée par le fait qu'elle contient en outre au moins un additif conventionnel choisi parmi un adoucissant, un conservateur, un agent séquestrant, un parfum, un agent épaississant, une huile, une silicone, un agent de cohésion, un agent alcalinisant ou acidifiant, un polymère hydrosoluble et une charge.

15. Composition de mascara selon la revendication 14, caractérisée par le fait que ledit agent épaississant est un dérivé cellulosique hydrosoluble choisi parmi les méthylcelluloses, les hydroxyéthylcelluloses, les hydroxypropylméthylcelluloses, les carboxyméthylcelluloses et leurs mélanges.

## Claims

1. Mascara composition, characterized in that it contains, as a mixture:
a) a pseudolatex composed of particles, with a mean diameter of between 10 and 300 nm, of a film-forming polymer derived from cellulose, insoluble in water, chosen from cellulose ethers, non-ionic cellulose esters and anionic cellulose esters containing carboxylic acid functional groups, the said carboxylic acid functional groups being neutralized to a degree of neutralization of between 10 and 80% using a non-volatile basic agent, and
b) at least one wax having a melting point of between 60°C and 110°C, and preferably between 65°C and 100°C.

2. Mascara composition according to Claim 1, characterized in that it contains from 0.8 to 20% by weight of dry matter of the pseudolatex and from 2 to 40% by weight of at least one wax with respect to the total weight of the said composition.

3. Mascara composition according to Claim 1 or 2, characterized in that the ratio by weight of the pseudolatex, expressed as weight of dry matter, to the wax is between 0.025 and 2.

4. Mascara composition according to any one of the preceding claims, characterized in that the film-forming polymer has an average molecular weight of between 2,000 and 700,000 and preferably of between 5,000 and 500,000.

5. Mascara composition according to any one of the preceding claims, characterized in that the said film-forming polymer is a cellulose ether chosen from ethyl-celluloses.

6. Mascara composition according to any one of Claims 1 to 4, characterized in that the said film-forming polymer is a non-ionic cellulose ester chosen from cellulose acetates, cellulose propionates, cellulose butyrates, cellulose acetopropionates and cellulose acetobutyrates.

7. Mascara composition according to any one of Claims 1 to 4, characterized in that the said film-forming polymer is an anionic cellulose ester containing carboxylic acid functional groups chosen from cellulose acetophthalate, cellulose acetate succinate, cellulose propionate succinate, cellulose butyrate succinate, cellulose acetopropionate succinate, cellulose acetobutyrate succinate, cellulose acetate trimellitate, cellulose propionate trimellitate, cellulose butyrate trimellitate, cellulose acetopropionate trimellitate and cellulose acetobutyrate trimellitate.

8. Mascara composition according to any one of Claims 1 to 4 and 7, characterized in that the carboxylic acid functional groups of the anionic cellulose ester are neutralized using a non-volatile basic agent chosen from: sodium hydroxide, potassium hydroxide, 2-amino-2-methyl-1-propanol, triethanolamine, triisopropanolamine, monoethanolamine, diethanolamine, tri(2-hydroxy-1-propyl)amine, 2-amino-2-methyl-1,3-propanediol and 2-amino-2-hydroxymethyl-1,3-propanediol.

9. Mascara composition according to any one of the preceding claims, characterized in that the pseudolatex contains a plasticizing agent in a proportion of between 5 and 90% by weight with respect to the weight of the film-forming polymer, the said plasticizing agent being distributed according to its partition coefficient between the particles and the aqueous phase of the pseudolatex.

10. Mascara composition according to any one of the preceding claims, characterized in that the said wax is chosen from the group consisting of beeswax, lanolin wax, Chinese insect wax, rice wax, carnauba wax, candelilla wax, ouricury wax, cork fibre wax, sugar cane wax, Japan wax, sumach wax, montan wax, microcrystalline waxes, paraffins, ozocerite, polyethylene waxes and hydrogenated oils.

11. Mascara composition according to any one of the preceding claims, characterized in that it additionally contains pigments in a proportion of between 3 and 25% by weight with respect to the total weight of the composition.

12. Mascara composition according to any one of the preceding claims, characterized in that it is provided in the form of an oil-in-water or water-in-oil emulsion or of a suspension.

13. Mascara composition according to Claim 12, characterized in that it is provided in the form of an emulsion containing at least one anionic or non-ionic surface-active agent in a proportion of between 2 and 30% by weight with respect to the total weight of the composition.

14. Mascara composition according to any one of the preceding claims, characterized in that it additionally contains at least one conventional additive chosen from an emollient, a preserving agent, a sequestering agent, a fragrance, a thickening agent, an oil, a silicone, a cohesion agent, a basifying or acidifying agent, a water-soluble polymer and a filler.

15. Mascara composition according to Claim 14, characterized in that the said thickening agent is a water-soluble cellulose derivate chosen from methylcelluloses, hydroxyethylcelluloses, hydroxypropylmethylcelluloses, carboxymethylcelluloses and their mixtures.

## Patentansprüche

1. Maskarazubereitung, dadurch gekennzeichnet, daß sie als Mischung enthält:
a) einen Pseudo-Latex, bestehend aus Teilchen, deren mittlerer Durchmesser zwischen 10 und 300 nm liegt, aus einem von Cellulose abgeleiteten, filmbildenden Polymer, das in Wasser unlöslich ist und ausgewählt ist unter Celluloseethern, nicht-ionischen Celluloseestern und anionischen Celluloseestern mit Carbonsäurefunktionen, wobei diese Carbonsäurefunktionen mit Hilfe eines basischen, nicht flüchtigen Mittels zu einem Neutralisierungsgrad zwischen 10 und 80 % neutralisiert sind, und
b) mindestens ein Wachs mit einem Schmelzpunkt zwischen 60 °C und 110 °C und vorzugsweise zwischen 65°C und 100 °C.

2. Maskarazubereitung gemäß Anspruch 1, dadurch gekennzeichnet, daß sie 0,8 bis 20 Gew.-% Trockenmaterial aus Pseudo-Latex und 2 bis 40 Gew.-% mindestens eines Wachses, bezogen auf das Gesamtgewicht der Zubereitung, enthält.

3. Maskarazubereitung gemäß den Ansprüchen 1 oder 2, dadurch gekennzeichnet, daß das Gewichtsverhältnis zwischen dem Pseudo-Latex, ausgedrückt als Gewicht des Trockenmaterials, und dem Wachs zwischen 0,025 und 2 liegt.

4. Maskarazubereitung gemäß einem der vorstehenden Ansprüche, dadurch gekennzeichnet, daß das filmbildende Polymer ein mittleres Molekulargewicht zwischen 2.000 und 700.000 und vorzugsweise zwischen 5.000 und 500.000 aufweist.

5. Maskarazubereitung gemäß einem der vorstehenden Ansprüche, dadurch gekennzeichnet, daß das filmbildende Polymer ein unter den Ethylcellulosen ausgewählter Celluloseether ist.

6. Maskarazubereitung gemäß einem der Ansprüche 1 bis 4, dadurch gekennzeichnet, daß das filmbildende Polymer ein nicht-ionischer Celluloseester ist, der unter den Celluloseacetaten, Cellulosepropionaten, Cellulosebutyraten, den Celluloseacetopropionaten und Celluloseacetobutyraten ausgewählt ist.

7. Maskarazubereitung gemäß einem der Ansprüche 1 bis 4, dadurch gekennzeichnet, daß das filmbildende Polymer ein anionischer Celluloseester mit Carbonsäurefunktionen ist, der unter dem Celluloseacetophthalat, dem Celluloseacetylsuccinat, dem Cellulosepropionylsuccinat, dem Cellulosebutyrylsuccinat, dem Celluloseacetopropionylsuccinat, dem Celluloseacetobutyrylsuccinat, dem Celluloseacetyltrimellitat, dem Cellulosepropionyltrimellitat, dem Cellulosebutyryltrimellitat, dem Celluloseacetopropionyltrimellitat und dem Celluloseacetobutyryltrimellitat ausgewählt ist.

8. Maskarazubereitung gemäß einem der Ansprüche 1 bis 4 und 7, dadurch gekennzeichnet, daß die Carbonsäurefunktionalitäten des anionischen Celluloseesters mit Hilfe eines basischen, nicht flüchtigen Mittels neutralisiert werden, das ausgewählt ist unter Natriumhydroxid, Kaliumhydroxid, 2 Amino-2-methyl-1-propanol, Triethanolamin, Triisopropanolamin, Monoethanolamin, Diethanolamin, Tri-((2-hydroxy)-1-propyl)-amin, 2-Amino-2-methyl-1,3-propandiol und 2-Amino-2-hydroxymethyl-1,3-propandiol.

9. Maskarazubereitung gemäß einem der vorstehenden Ansprüche, dadurch gekennzeichnet, daß der Pseudo-Latex einen Weichmacher in einer Menge von 5 bis 90 Gew.-% enthält, bezogen auf das Gewicht des filmbildenden Polymeren, wobei der Weichmacher gemäß seines Verteilungscoeffizienten zwischen den Teilchen und der wässrigen Phase des Pseudo-Latex verteilt ist.

10. Maskarazubereitung gemäß einem der vorstehenden Ansprüche, dadurch gekennzeichnet, daß das Wachs aus der aus Bienenwachs, Lanolinwachs, chinesischem Insektenwachs (Chinawachs), Reiswachs, Carnaubawachs, Candelillawachs, Ouricury-Wachs, Hanffaserwachs, Zuckerrohrwachs, Japanwachs, Sumachwachs, mikrokristallinen Wachsen, Paraffin, Ozokerit, Polyethylenwachsen und hydrierten Ölen ausgewählt ist.

11. Maskarazubereitung gemäß einem der vorstehenden Ansprüche, dadurch gekennzeichnet, daß sie zusätztlich Pigmente in Mengen von 3 bis 25 Gew. % enthält, bezogen auf das Gesamtgewicht der Zubereitung.

12. Maskarazubereitung gemäß einem der vorstehenden Ansprüche, dadurch gekennzeichnet, daß sie in Form einer Öl-in-Wasser-Emulsion oder einer Wasser-in-Öl-Emulsion oder einer Suspension vorliegt.

13. Maskarazubereitung gemäß Anspruch 12, dadurch gekennzeichnet, daß sie in Form einer Emulsion vorliegt, die mindestens ein anionisches oder nichtionisches oberflächenaktives Mittel in einer Menge zwischen 2 und 30 Gew.-% enthält, bezogen auf das Gesamtgewicht der Zubereitung.

14. Maskarazubereitung gemäß einem der vorstehenden Ansprüche, dadurch gekennzeichnet, daß sie zusätztlich mindestens ein herkömmliches Additiv enthält, das ausgewählt ist unter einem Weichmacher, einem Konservierungsmittel, einem Bindemittel, einem Parfüm, einem Verdickungsmittel, einem Öl, einem Silikon, einem Kohäsionsmittel, einem Säuerungsmittel oder einem basifizierenden Mittel, einem wasserlöslichen Polymer oder einem Zuschlag.

15. Maskarazubereitung gemäß Anspruch 14, dadurch gekennzeichnet, daß das Verdickungsmittel ein wasserlösliches Cellulosederivat ist, das ausgewählt ist unter Methylcellulosen, Hydroxyethylcellulosen, Hydroxypropylmethylcellulosen, Carboxymethylcellulosen und deren Mischungen.
